# EUROPÄISCHE PATENTSCHRIFT

(11) **EP 0 714 630 B1**
(45) Veröffentlichungstag und Bekanntmachung des Hinweises auf die Patenterteilung: **02.04.2003**
(21) Anmeldenummer: 95850197.5
(22) Anmeldetag: 08.11.1995
(51) Int. Cl.: A61B 5/0408, A61B 5/00

(54) **Verfahren zur Herstellung einer Sensorelektrode**
Manufacturing method for a sensor electrode
Procédé de fabrication d'un capteur à électrode

(30) Priorität: 10.11.1994 DE 4440224
(43) Veröffentlichungstag der Anmeldung: 05.06.1996
(73) Patentinhaber: St. Jude Medical AB, 175 84 Järfälla (SE)
(72) Erfinder: Mund, Konrad, D-91080 Uttenreuth (DE); Rao, J. Raghavendra, D-91058 Erlangen (DE); Datz, Armin, D-91099 Poxdorf (DE)

(56) Entgegenhaltungen:
- EP-A- 0 116 280
- EP-A- 0 170 998
- EP-A- 0 455 071
- WO-A-95/11723
- DE-A- 3 313 977
- FR-A- 2 345 169
- FR-A- 2 345 170

## Beschreibung

Die Erfindung betrifft ein Verfahren zur Herstellung einer Arbeitselektrode für implantierbare elektrochemische Sauerstoffsensoren.

Bei physiologisch gesteuerten Herzschrittmachern dient insbesondere die Sauerstoffkonzentration im Blut bzw. Gewebe als Steuerparameter. Zur Messung der Sauerstoffkonzentration hat sich ein implantierbarer elektrochemischer Sauerstoffsensor als vorteilhaft erwiesen, dessen Arbeitselektrode eine Glaskohlenstoff-Elektrode mit glatter Oberfläche ist (siehe: EP 0 170 998 A2). Diese Arbeitselektrode wird - zum Reduzieren des im Elektrolyt gelösten Sauerstoffs - für kurze Zeit (etwa 5 bis 50 ms) beispielsweise auf ein Potential von -1 V (gegen Ag/AgCl) potentiostatiert und kehrt dann wieder auf das Ruhepotential (ca. 0 V) zurück. Von Vorteil ist dabei, daß die Arbeitselektrode nicht mit einer Membran versehen werden muß, weil nämlich der Glaskohlenstoff für die Oxidation von im Elektrolyt vorhandenen Substanzen elektrokatalytisch inert ist und nur die Sauerstoffreduktion katalysiert.

Ein Sauerstoffsensor der vorstehend genannten Art besteht bislang im allgemeinen aus einer 2- oder 3-Elektrodenanordnung, wobei die Elektroden in Form eines Katheters zusammengebaut sind. Die Arbeitselektrode (aus Glaskohlenstoff) ist dabei halbkugelförmig, ausgestaltet und bildet die Katheterspitze. Von Vorteil ist es jedoch, wenn der Sauerstoffsensor in das Elektrodenkabel des Herzschrittmachers integriert werden kann. Dazu muß die Arbeitselektrode eine ringförmige Gestalt aufweisen, wie in der EP-A-0 455 071 beschrieben.

Aufgabe der Erfindung ist es , ein optimiertes Verfahren anzugeben, daß die Herstellung einer ringförmigen Arbeitselektrode für implantierbare elektrochemische Sauerstoffsensoren erlaubt.

Dies wird erfindungsgemäß durch ein Verfahren erreicht, bei dem auf einen Ring oder Hohlzylinder aus biokompatiblem, inertem, elektrisch leitfähigem Material eine dünne Schicht eines homogenen Gemisches aus einem Harz auf Epoxidbasis, einem Härter und pulverförmigem Glaskohlenstoff aufgebracht und das Harz gehärtet wird.

Bei der nach diesem Verfahren hergestellten Arbeitselektrode besteht die wirksame Schicht aus Glaskohlenstoffpulver, das porenfrei in eine Kunststoffmatrix eingebettet ist. Die katalytische Aktivität und die elektrochemische Doppelschichtkapazität dieser Schicht sind gering. Die Schicht ist außerdem mechanisch stabil sowie elektrisch leitend und kann maschinell gut bearbeitet werden. Durch eine mechanische Bearbeitung, die beispielsweise durch Drehen erfolgen kann, wird Glaskohlenstoff freigelegt, was für die elektrische Leitfähigkeit vorteilhaft ist. Die Dicke der gehärteten Schicht beträgt vorzugsweise ≥ 250 µm. Im übrigen führten Versuche, Ringe oder Hohlzylinder unmittelbar aus Glaskohlenstoff anzufertigen, zu Körpern mit einer unzureichenden mechanischen Stabilität.

Zur Herstellung der Arbeitselektrode nach der Erfindung wird das Glaskohlenstoffpulver vorteilhaft in einer solchen Konzentration in das Harz eingebracht, daß gewährleistet ist, daß das gehärtete Material eine elektronische Leitfähigkeit aufweist, die nicht kleiner als 2 mS/cm ist. Vorzugsweise beträgt der Glaskohlenstoff-Gehalt des Gemisches aus Harz, Härter und Glaskohlenstoff ≥ 50 Gew.-%. Das Glaskohlenstoffpulver weist vorteilhaft einen Korndurchmesser zwischen 1 und 100 µm auf, vorzugsweise zwischen 10 und 50 µm. Sphärische Partikel haben sich dabei als besonders geeignet erwiesen; es kann aber auch splittriges Material eingesetzt werden. Glaskohlenstoff ist im übrigen ein körperverträgliches Material.

Beim Verfahren nach der Erfindung werden Harze auf der Basis von Epoxiden eingesetzt. Dies sind beispielsweise gängige Epoxidharze, insbesondere auf der Grundlage von Bisphenolen. Epoxidharze werden - in an sich bekannter Weise - mit geeigneten Härtern in duroplastische Kunststoffe übergeführt, d.h. gehärtet. Dies kann bei Raumtemperatur oder bei erhöhter Temperatur erfolgen. Geeignete Härter sind insbesondere polyfunktionelle aliphatische oder aromatische Amine und Säureanhydride.

Als besonders geeignet haben sich sogenannte EP/IC-Harze erwiesen, d.h. Harze auf Epoxid/Isocyanat-Basis, die ebenfalls in an sich bekannter Weise gehärtet werden (siehe dazu beispielsweise EP 0 129 787 B1 und EP 0 130 454 B1). Kunststoffe auf Epoxidharzbasis sind im übrigen bioverträglich und biostabil, d.h. im Körper erfolgt keine Auflösung und keine Degradation.

Die beim Verfahren nach der Erfindung eingesetzten Ringe bzw. Hohlzylinder bestehen aus biokompatiblem, inertem Material. Dafür eignen sich insbesondere Metalle, vorzugsweise Titan, Platin und Gold. Vorteilhaft können auch Elektrodenkörper aus Titan eingesetzt werden, die mit einer Schicht aus dichtem Titannitrid (TiN) versehen sind. Titannitrid ist leitfähiger als reines Titan, ebenfalls inert gegenüber Sauerstoff und auch gut körperverträglich (siehe dazu beispielsweise EP 0 115 778 B1).

Anhand von Ausführungsbeispielen soll die Erfindung noch näher erläutert werden.

### Beispiel 1

15 g eines kommerziellen Epoxidharzes auf der Basis von Bisphenol A (mittleres Molekulargewicht < 700) und 1,5 g eines kommerziellen Härters auf der Basis von Triethylentetramin werden zusammen gemischt und 6 bis 8 min entgast. Zu dieser Mischung werden 18 g sphärisches Glaskohlenstoffpulver (Partikeldurchmesser: 20 bis 50 µm) gegeben, dann wird sorgfältig gemischt, bis eine homogene Masse erhalten wird; anschließend wird nochmals für 20 bis 30 min evakuiert.

Die viskose Masse wird auf einen Titanring mit einem Durchmesser von 2,2 mm und einer Breite von 1,1 mm aufgebracht und dann gehärtet. Die Härtung erfolgt entweder bei Raumtemperatur (über Nacht) oder bei 80°C (2 h). Die gehärtete Masse wird anschließend noch mechanisch bearbeitet, wobei eine gleichmäßige Schicht mit einer Dicke von ca. 300 um erhalten wird (Glaskohlenstoffgehalt: 52,2 %).

### Beispiel 2

11,6 g eines kommerziellen Epoxidharzes auf der Basis von Bisphenol A (mittleres Molekulargewicht < 350) und 8,4 g Diphenylmethandiisocyanat werden in einen 250 ml-Kolben gegeben und 10 min unter Rühren bei 60°C entgast. Nachfolgend werden 31 g sphärisches Glaskohlenstoffpulver (Partikeldurchmesser: 20 bis 30 µm) sorgfältig zugegeben, und dann wird nochmals für 2 bis 3 h unter Rühren bei derselben Temperatur evakuiert. Nach der vollständigen Entgasung werden 0,25 g eines substituierten Imidazols als Härtungskatalysator zugegeben.

Die viskose Masse wird auf einen Titanring (Durchmesser: 2,2 mm; Breite: 1,1 mm) aufgebracht, der zuvor durch Sputtern mit einer dünnen TiN-Schicht versehen wurde, dann wird bei 120°C (1 h) und 150°C (1 h) gehärtet. Die gehärtete Masse wird anschließend noch mechanisch bearbeitet, wobei eine gleichmäßige Schicht mit einer Dicke von ca. 300 µm erhalten wird (Glaskohlenstoffgehalt: 60,5 %).

Die auf die vorstehend beschriebene Weise hergestellten Elektroden haben einen spezifischen elektrischen Widerstand im Bereich zwischen 50 und 150 Ω·cm und weisen extrem niedrige Kapazitäten von < 20 µF/cm² bei einer Frequenz von 1 Hz auf. Diese Werte sind vergleichbar mit denjenigen von Elektroden aus reinem Glaskohlenstoff. Sauerstoffmessungen mittels Elektroden nach der Erfindung zeigen eine schnelle und deutliche Antwort auf Änderungen der Sauerstoffkonzentration. Die Empfindlichkeit beträgt im allgemeinen > 12 % bei einer Änderung von 0 auf 5 % Sauerstoff.

## Patentansprüche

1. Verfahren zur Herstellung einer Arbeitselektrode für implantierbare elektrochemische Sauerstoffsensoren, mit einem Ring oder Hohlzylinder aus biokompatiblem, inertem, elektrisch leitfähigem Material **dadurch gekennzeichnet, daß** auf den Ring oder Hohlzylinder eine dunne Schicht eines homogenen Gemisches aus einem Harz auf Epoxidbasis, einem Härter und pulverförmigem Glaskohlenstoff aufgebracht wird, und daß das Harz gehärtet wird.

2. Verfahren nach Anspruch 1, **dadurch gekennzeichnet, daß** die Oberfläche der gehärteten Schicht mechanisch bearbeitet wird.

3. Verfahren nach Anspruch 1 oder 2, **dadurch gekennzeichnet, daß** die Dicke der gehärteten Schicht ≥ 250 µm beträgt.

4. Verfahren nach einem der Ansprüche 1 bis 3, **dadurch gekennzeichnet, daß** die gehärtete Schicht eine elektronische Leitfähigkeit ≥ 2 mS/cm besitzt.

5. Verfahren nach einem oder mehreren der Ansprüche 1 bis 4, **dadurch gekennzeichnet, daß** der Gehalt des Gemisches an Glaskohlenstoff ≥ 50 Gew.-% beträgt.

6. Verfahren nach einem oder mehreren der Ansprüche 1 bis 5, **dadurch gekennzeichnet, daß** das Glaskohlenstoffpulver eine Korngröße zwischen 1 und 100 µm aufweist, vorzugsweise zwischen 10 und 50 µm.

7. Verfahren nach einem oder mehreren der Ansprüche 1 bis 6, **dadurch gekennzeichnet, daß** ein Harz auf Epoxid/Isocyanat-Basis verwendet wird.

8. Verfahren nach einem oder mehreren der Ansprüche 1 bis 7, **dadurch gekennzeichnet, daß** der Ring bzw. Hohlzylinder aus Metall besteht.

9. Verfahren nach Anspruch 8, **dadurch gekennzeichnet, daß** das Metall Titan, Platin oder Gold ist.

10. Verfahren nach Anspruch 9, **dadurch gekennzeichnet, daß** das Titan mit Titannitrid beschichtet ist.

## Claims

1. Method of producing a working electrode for implantable electrochemical oxygen sensors, with a ring or hollow cylinder made from biocompatible, inert, electrically conductive material, **characterised in that** a thin layer of a homogeneous mixture of an epoxy-based resin, a hardener and powdered glass carbon is applied to the ring or hollow cylinder, and that the resin is hardened.

2. Method as claimed in Claim 1, **characterised in that** the surface of the hardened layer is mechanically processed.

3. Method as claimed in Claim 1 or 2, **characterised in that** the thickness of the hardened layer is ? 250 µm

4. Method as claimed in one of Claims 1 to 3, **characterised in that** the hardened layer has an electronic conductivity = 2 mS/cm.

5. Method as claimed in one or more of Claims 1 to 4, **characterised in that** the glass carbon content of the mixture is = 50% by weight.

6. Method as claimed in one or more of Claims 1 to 5, **characterised in that** the glass carbon powder has a particle size of between 1 and 100 µm, preferably between 10 and 50 µm.

7. Method as claimed in one or more of Claims 1 to 6, **characterised in that** an epoxy/isocyanate-based resin is used.

8. Method as claimed in one or more of Claims 1 to 7, **characterised in that** the ring or hollow cylinder is made from metal.

9. Method as claimed in Claim 8, **characterised in that** the metal is titanium, platinum or gold.

10. Method as claimed in that the titanium is coated with titanium nitride.

## Revendications

1. Procédé de fabrication d'une électrode de travail pour des capteurs d'oxygène électrochimiques implantables ayant un anneau ou un cylindre creux en matériau biocompatible, inerte et conducteur de l'électricité, **caractérisé en ce que** l'on dépose sur l'anneau ou sur le cylindre creux une couche mince d'un mélange homogène constitué d'une résine à base d'époxyde, d'un durcisseur et d'un carbone vitreux pulvérulent et **en ce que** l'on durcit la résine.

2. Procédé suivant la revendication 1, **caractérisé en ce que** l'on usine mécaniquement la surface de la couche durcie.

3. Procédé suivant la revendication 1 ou 2, **caractérisé en ce que** l'épaisseur de la couche durcie est ≥ 250 µm.

4. Procédé suivant l'une des revendications 1 à 3, **caractérisé en ce que** la couche durcie a une conductivité électronique ≥ 2 mS/cm.

5. Procédé suivant l'une ou plusieurs des revendications 1 à 4, **caractérisé en ce que** la teneur du mélange en carbone vitreux est ≥ 50 % en poids.

6. Procédé suivant l'une ou plusieurs des revendications 1 à 5, **caractérisé en ce que** la poudre de carbone vitreux a une granulométrie comprise entre 1 et 100 µm, de préférence comprise entre 10 et 50 µm.

7. Procédé suivant l'une ou plusieurs des revendications 1 à 6, **caractérisé en ce que** l'on utilise une résine à base d'époxyde/isocyanate.

8. Procédé suivant l'une ou plusieurs des revendications 1 à 7, **caractérisé en ce que** l'anneau ou le cylindre creux est en métal.

9. Procédé suivant la revendication 8, **caractérisé en ce que** le métal est du titane, du platine ou de l'or.

10. Procédé suivant la revendication 9, **caractérisé en ce que** le titane est revêtu de nitrure de titane.
